# EUROPEAN PATENT SPECIFICATION

(11) **EP 3 456 188 B1**
(45) Date of publication and mention of the grant of the patent: **09.06.2021**
(21) Application number: 17795786.7
(22) Date of filing: 20.02.2017
(51) Int. Cl.: A01K 23/00

(54) **ABSORBENT ARTICLE FOR PET**
SAUGFÄHIGER ARTIKEL FÜR HAUSTIERE
ARTICLE ABSORBANT POUR ANIMAL DE COMPAGNIE

(30) Priority: 11.05.2016 JP 2016095408
(43) Date of publication of application: 20.03.2019
(73) Proprietor: Unicharm Corporation, Ehime 799-0111 (JP)
(72) Inventor: KOMATSUBARA, Daisuke, Kanonji-shi, Kagawa 769-1602 (JP)
(74) Representative: Staeger & Sperling Partnerschaftsgesellschaft mbB
(86) International application number: PCT/JP2017/006037
(87) International publication number: WO 2017/195425

(56) References cited:
- JP-A- 2009 125 087
- JP-A- 2012 205 560
- JP-A- 2014 117 179
- JP-A- 2016 042 793

## Description

### [Technical Field]

The present disclosure relates to absorbent articles used to collect and contain body exudates of pet animals (sometimes designated simply as 'pet') such as canines and felines.

### [Background Art]

Conventionally, absorbent articles for pet animals such as canines and felines are well known. For example, Patent Literature 1 discloses an absorbent article of open-type having a fastening system for pet animals. The article includes a ventral region, a dorsal region, a liquid permeable interior sheet, a liquid impermeable exterior sheet and an absorbent core interposed between the interior and exterior sheets wherein the article is worn on a pet by encircling its body.

### [Prior Art Document]

### [Patent Literature]

Patent Literature 1: unexamined patent application publication (JP2013-9657 A)
Further prior art in this technical field is disclosed in documents JP 2014-117179 A and JP 2009-125087 A.

### [Summary of Invention]

### [Technical Problem]

A pet such as a dog has less constricted part in the belly region, and an absorbent article worn on the pet may cause a position displacement by the movements including the pet's walking. When the article has caused the position displacement in a state covering the external excretory organ of the pet, body exudates such as urine may leak out.

A dog such as a pet moves the forearms and the hind legs only in the front-rear direction when walking. When article is worn on the dog, part of the article being positioned at the high thighs and its vicinities, When the dog moves the hind legs, the part of the article abuts against the high thighs and its vicinities, thereby interfering with the walking and reducing an amount of the movements.

An object of the present invention disclosed is to improve an absorbent article for a pet animal and to provide the article that has fittability capable of preventing the article from causing the position displacement relative to the pet and that does not interfere with the walking.

### [Solution to Problem]

To achieve the above object, the present invention is defined by the features according to claim 1 and has a longitudinal direction and a lateral direction, and includes a ventral region, a dorsal region, an intermediate region extending in the longitudinal direction between the ventral and dorsal regions, and an absorbent core extending between the front and intermediate regions.

According to the absorbent article for a pet animal, the ventral region and the dorsal region each have both lateral edges extending in the longitudinal direction, an lower edge and an upper edge both extending in the lateral direction, an lower edge area having the lower edge, an upper edge area having the upper edge, a middle area positioned between the lower edge area and the upper edge area, and a connecting means for detachably connect both the lateral edges of the ventral region and both the lateral edges of the dorsal region to each other. The intermediate region includes a tail opening passable through a tail of the pet animal. The absorbent core is positioned between the tail opening and the ventral region. The lower edge area is defined by an elastic area contractible in the lateral direction.

The absorbent article may include a rectangular ventral panel forming the ventral region, a rectangular dorsal panel forming the dorsal region, and a rectangular absorbent panel including an absorbent core and extending in the longitudinal direction while connecting to the ventral region and the dorsal region. The lower edge of the ventral region extends linearly in the lateral direction, both lateral edges of the intermediate region extend in the longitudinal direction, and both lateral edges of the intermediate region and the lower edge of the ventral region orthogonally intersect with each other. With such embodiment, the ventral panel and the dorsal panel are not positioned outside of the upper upper thighs of the hind legs 7, and when the hind legs has moved back and forth, the pet's walking may not be interfered by butting of part of the absorbent article against the hind legs.

The absorbent article has a body facing surface to face a pet animal's body and a body non-facing surface opposite to the body facing surface. The ventral region includes an interior sheet lying on the body facing surface, an exterior sheet lying on the body non-facing surface, and thread-like or string-like elastic members extending in the lateral direction between the interior sheet and the exterior sheet. The elastic area is a first elastic area defined by the elastic members, and the upper edge area is a second elastic area defined by the elastic members. A tensile stress of the first elastic area is stronger than a tensile stress of the second elastic area. With such embodiment, although the pet animal does not have large constricted part on its body, the absorbent article put on the pet animal can reliably be prevented from displacing the position by the tensile stress of the second elastic area. Moreover, the tensile stress of the first elastic area is stronger than the tensile stress of the second elastic area, and even when the pet has moved back and forth during its walking, the lower edge area of the ventral region can stably be abutted against the pet's body by the tensile stress of the first elastic area. In addition, even if the hind legs have abutted against the lower edge area during the pet's walking, the lower edge area having elasticity, the lower area is deformable in response to the movements of the hind legs so as not to give a discomfort feeling against the pet animal.

A loin opening and a pair of leg openings are respectively defined when both lateral edges of the ventral region and both lateral edges of the dorsal region has been connected to each other. The ventral region has the first elastic area at the side of the leg openings and the second elastic area at the side of the loin opening. The first elastic area is defined by both elastic areas spaced apart from each other in the lateral direction, and an inelastic area is defined by an area overlapping with the absorbent core in planar view between both elastic areas. The middle area is defined by a third elastic area adjacent to the first and second elastic areas and contractible in the lateral direction. With such embodiment, the inelastic area being defined at an area overlapped with the absorbent core, absorption performance of the absorbent core should not be inhibited by gathering due to the contractile force of the first elastic area.

The connecting means include a pair of fastening tabs extending outward in the lateral direction from both lateral edges of the ventral region and receiving areas positioned on the body non-facing surface of the dorsal region and releasably engageable with the respective engageable areas. The pair of fastening tabs may each be disposed nearer to the upper edge than the lower edge of the ventral region. With such embodiment, it is possible to prevent the upper thighs of the hind legs from being excessively tightened by the fastening tabs. In addition, peripheries of the upper thighs of the hind legs should not be excessively tightened by the lower edge area of the ventral region. Thus, even when the lower edge area has abutted against the hind legs during the pet's walking, the lower edge area is deformable in response to the movements of the hind leg and should not create a discomfort feeling against the pet animal.

The fastening tabs may each have a fixed end fixed to the ventral region and a free end extending in the lateral direction without being fixed to the ventral region. The ventral region may have a second inelastic area at both lateral side in the lateral direction of the first elastic area. The end may be positioned to the second inelastic area. With such embodiment, the fixing of the proximal end and the ventral region can be ensured.

The dorsal region may have an elastic area contractible in the lateral direction. With such embodiment, the absorbent article can be abutted against the pet's body also in the dorsal region, and an area of the absorbent area to be abutted against the pet animal can be enlarged and the absorbent article put on the pet animal can reliably be prevented from displacing the position,

### [Advantageous Effects of Invention]

With the absorbent article for a pet animal according to one or more embodiments of the present invention, the lower edge area is defined by the elastic area contractible in the lateral direction, and the absorbent article has fittability to abut the ventral region against the pet animal. Thus, the absorbent article put on the pet animal can be inhibited from displacing the position. In addition, the lower edge area being defined by the elastic area contractible in the lateral direction, the lower edge area can be abutted against the pet animal such that the lower edge area does not cause the position displacement and the pet animal's walking should not be interfered.

### [Brief Description of Drawings]

The drawings illustrate specific embodiments of the present invention including optional and preferred embodiments as well as essential features of the invention.
Fig. 1 is a perspective view illustrating a diaper put on a dog as one example of the absorbent article for a pet animal according to a first embodiment of the present invention.
Fig. 2 is a partially cutaway plan view of the diaper flat-opened up to respective elastic members have been stretched in vertical and lateral directions to the respective maximum elongations (to the extent that gathers under contractions of the respective elastic members substantially disappear).
Fig. 3 is a schematic cross-sectional view taken along line III-III in Fig. 2.
Fig. 4 is an enlarged view of a ventral region in Fig. 2.
Fig. 5 is a side view of the dog wearing the diaper partially enlarged area encircled by a dot-dash line in Fig. 2.
Fig. 6 is a perspective view of the diaper according to a second embodiment of the present invention.

### [Description of Embodiments]

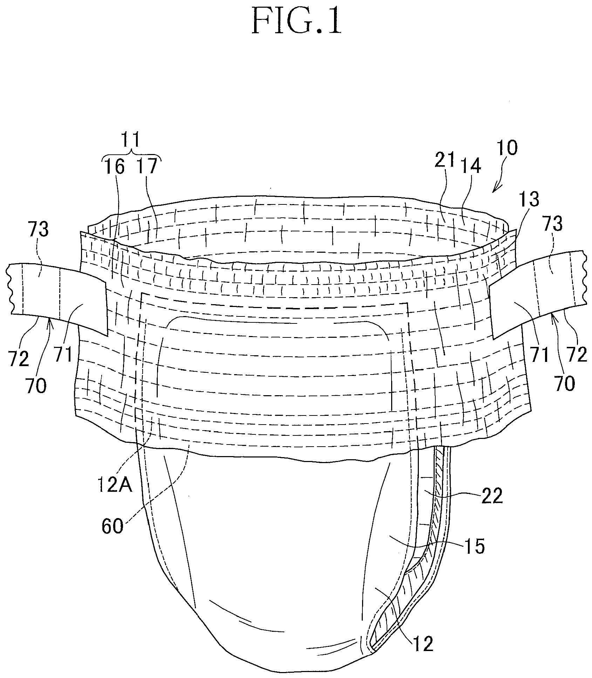

The embodiments described below relate to a diaper for a dog as one example of an absorbent article for a pet animal as illustrated in Figs. 1 to 6, including both optional and preferred features as well as those features which are essential features of the present invention.

### <First Embodiment>

Referring to Figs. 1 to 5, a diaper 10 for a dog as one example of an absorbent article for a pet animal may have a longitudinal direction Y, a lateral direction X, a thickness direction Z, a longitudinal axis P bisecting a dimension in the lateral direction X, a lateral axis Q bisecting a dimension in the longitudinal direction Y, a body facing surface body facing the body of the dog, and a body non-facing surface opposite to the body facing surface.

The diaper 10 may include a waist panel 11 that assumes an annular shape when put on, an absorbent panel 12 attached on the body facing surface of the waist panel 11, a ventral region 13, a dorsal region 14, and an intermediate region 15 extending between the ventral and dorsal regions 13, 14. The waist panel 11 may include a ventral panel 16 lying on the ventral region 13, and a dorsal panel 17 lying on the dorsal region 14.

The ventral panel 16 and the dorsal panel 17 may respectively intersect the absorbent panel 12 and have a laterally long rectangle defined by lower edges 16a, 17a extending in the lateral direction X, upper edges 16b, 17b opposite to the lower edges 16a, 17a, both lateral edges 16c, 17c extending between the lower edges 16a, 17a and the upper edges 16b, 17b.

The ventral panel 16 and the dorsal panel 17 may respectively include an interior sheets 25 lying on the body facing surface, and an exterior sheets 27 lying on the body non-facing surface. The interior and exterior sheets 25, 27 may be formed of liquid impermeable spunbonded-meltbrown-spunbonded fibrous nonwoven fabrics, spunboned fibrous nonwoven fabrics, plastic sheets, or laminated sheets of them, each having a mass in a range of 15 g/m² to 30 g/m². The interior and exterior sheets 25, 27 can be bonded to each other with hot melt adhesive applied to at least one of their facing surfaces, or by a heat welding means.

The ventral panel 16 and the dorsal panel 17 may have respectively thread-, strand- or string-like first elastic members 41 secured in a contractible manner under tension between the interior and exterior sheets 25, 27. The ventral panel 16 and the dorsal panel 17 are elasticized at least in the lateral direction X by the first elastic members 41. The interior and exterior sheets 25, 27 may be bonded to each other with only hot melt adhesive applied to approximately the entire peripheral surfaces of the first elastic members 41.

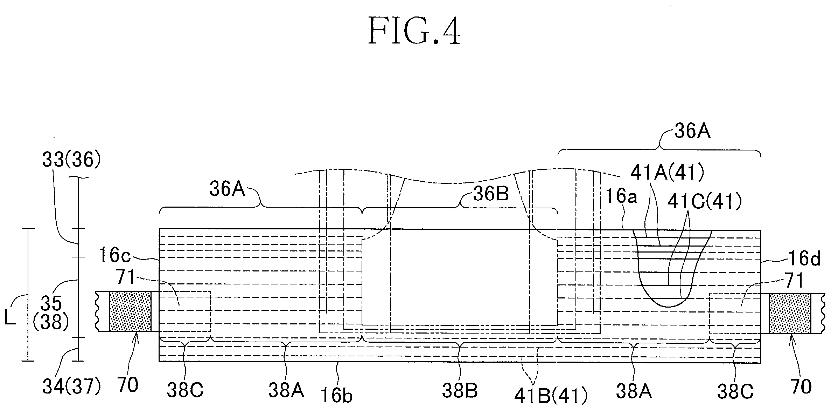

As shown in Fig. 4, the ventral region 16 may have an lower edge area 33 having the lower edge 16a, an exterior edge area 34 having the upper edge 16b, and a middle area 35 between the interior and exterior edge areas 33, 34. The lower edge area 33 may have a width of about 1/2 to about 1/5 of a longitudinal dimension L, the upper edge area 34 may have about 1/2 to about 1/5 of the longitudinal dimension L, and the middle area 35 may have about 1/2 to about 1/5 of the longitudinal dimension L. A description below will mainly be made about only the ventral panel 16. For the dorsal panel 17, its configuration being the same as that of the ventral panel 16, its description may hereinafter be omitted.

The first elastic members 41 may include more than one lower elastic member 41A extending in the lateral direction X along the lower edge 16a, more than one upper elastic member 41B extending in the lateral direction X along the upper edge 16b, extending in the lateral direction X along the upper edge 16b, and more than one middle elastic member 41c extending in the lateral direction between the lower and upper elastic members 41A, 41B. By the lower elastic member 41A, a first elastic area 36 contractible in the lateral direction X is defined in the lower edge area 33. Similarly, by the upper elastic member 41B, a second elastic area 37 contractible in the lateral direction is defined in the upper edge area 34. Similarly, by the middle elastic member 41C, a third elastic area 38 contractible in the lateral direction X is defined in the middle area 35. The middle area 35 is adjacent to the lower and upper edge area 33, 34.

The lower elastic member 41A and the middle elastic member 41C may extend between the respective lateral edges 16c, 16d of the ventral panel 16 and respective lateral edges 60c, 60d of an absorbent core 60 without overlapping with the absorbent core 60 in planar view(in a thickness direction orthogonal to the longitudinal and lateral directions Y, X), and the first elastic area 36 and the third elastic area 38 are respectively defined by both elastic areas 36A and both elastic areas 38A that are respectively spaced apart from each other in the lateral direction X, and an inelastic area 36B in which the first and middle elastic members 41A, 41C are not overlapped with the absorbent core 60 is defined between the respective lower and middle elastic members 41A, 41C. Thus, a contractile force of the lower and middle elastic members 41A, 41C does not directly act on the absorbent core 60, and the absorbent core 60 can be prevented from being contracted under the contractile force of the lower and middle elastic members 41A, 41C and from gathering and twisting thereby.

The lower, upper and middle elastic members 41A, 41B, 41C may respectively be composed of, for example, thread-, string- or strand-like elastic materials having fineness of about 300 dtex about 1200 dtex, and secured to the interior and exterior sheets 25, 27 in a contractible manner under tension at a stretch ratio in a range of about 1.5 to about 4.0.

As the lower, upper and middle elastic members 41A, 41B, 41C may be formed of, for example, elastic materials having the same fineness may be used. In these elastic members, a longitudinal pitch of the lower elastic member 41A may be smaller than that of the upper elastic member 41B, and a tensile stress of the first elastic area 36 may be stronger than that of the second elastic area 37; a longitudinal pitch of the upper elastic member 41B may be smaller than that of the middle elastic member 41C, and a tensile stress of the second elastic area 37 may be stronger than that of the third elastic area 38.

The absorbent panel 12 may have an overall rectangular shape defined by a front end attached on the body facing surface of the ventral panel 16, a rear end 12B attached on the body facing surface of the dorsal panel 17, and an intermediate area extending in the longitudinal direction Y between the front and rear ends 12A, 12B. The absorbent panel 12 may include a cover sheet 50, an absorbent core 60 disposed on the cover sheet 50, a bodyside liner 61 covering the body facing surface of the absorbent core 60, formed of a liquid permeable sheet, and a liquid barrier sheet 61 interposed between the cover sheet 50 and the absorbent core 60.

The absorbent core 60 is defined by a front end edge 60a, a rear end edge 60b and both convex lateral edges 60c, 60d, and may be formed of a mixture of superabsorbent particles having a water absorption capacity at least 10 times as high as own mass thereof, wood fluff pulp, and optionally a small amount of thermoplastic fibers. The absorbent core 60 may be covered with a core wrapping sheet (not shown) formed of hydrophilic and liquid diffusible fibrous nonwoven fabrics or tissue paper. The core wrapping sheet may be bonded to the bodyside liner 61 and/or the liquid barrier sheet 62 with hot melt adhesive.

The cove sheet 50 may have a middle area 51 disposed with the absorbent core 60 and containment flaps 52 positioned on both lateral sides of the middle area 51. The containment flaps 52 may each be folded inward in the lateral direction X, and may have both longitudinal ends, both proximal edges 55 fixed to the middle area 51 and the liquid barrier sheet 62 while extending in the longitudinal direction between both the longitudinal ends, both distal edges 56 positioned inside of both proximal edges 55 in the lateral direction. Each distal edge 56 may be positioned outside of the both proximal edge 55 in the lateral direction. Each distal edge 56 may have a sleeve-like edge 56a in which a flap elastic member 58 may be secured in a contractible condition under tension. In a worn condition of the diaper 10, the distal edge 56 can be spaced away from the bodyside liner 61 toward the dog's body under contraction of the flap elastic member 58 so that the distal edge 56 can come in contact with the dog' s body, thereby forming a barrier for preventing body exudates from leaking sideways.

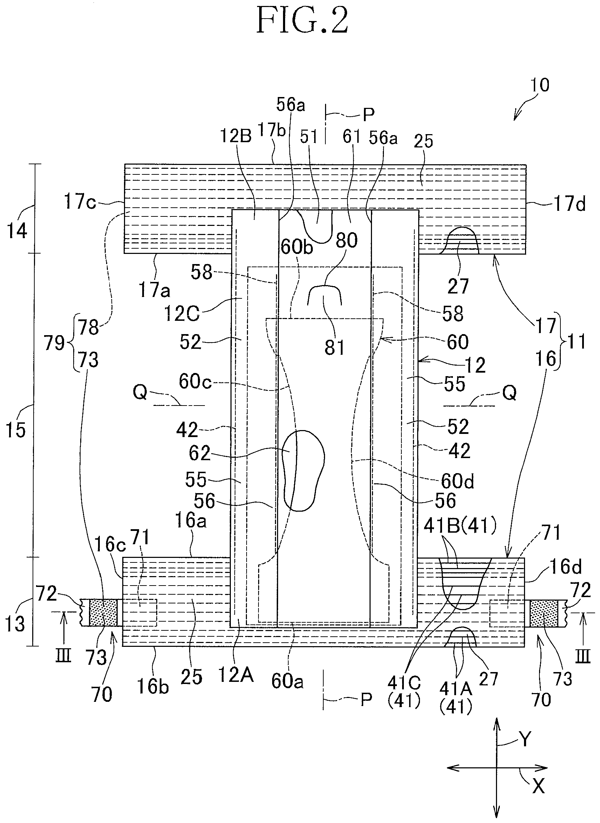

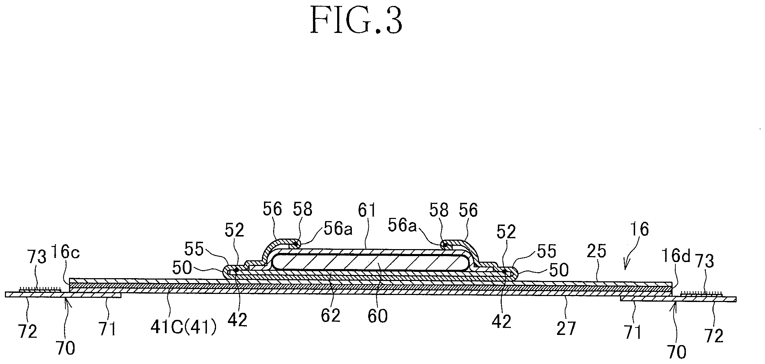

Referring to Figs. 2 and 3, second elastic members 42 extending in the longitudinal direction Y may each be secured in a contractible condition under tension to each proximal edge 55 of the containment flaps 52 that is positioned outward of both lateral edges 60c, 60d of the absorbent core 60 in the lateral direction X whereby lateral elastic areas contractible in the longitudinal direction Y on which that a contractile force of the second elastic members 42 acts can be formed at the containment flaps 52. This makes it possible to fit the containment flaps 52 to the upper thighs of the hind legs 7 and to prevent body exudates of the dog 5 from leaking sideways due to the position displacement of the lateral elastic areas.

Referring to Figs. 1 and 2, a pair of fastening tabs 70 may each have a fixed part 71 attached on the exterior surface of each lateral edge of the ventral region 13. The fastening tabs 70 may each have a free part 72 contiguous to the fixed part 71 and extending from lateral edges 16c, 16d outward in the lateral direction X, and the free part 72 may have an engageable area 73 on its interior surface. The engageable area 73 may have multiple fastening elements composed of hooks of a mechanical fastener that are releasably engageable with the exterior surface of the dorsal region 14 wherein the fastening elements are provided on a base sheet of the fastening tab 70.

The middle elastic members 41c may be non-elasticized on a site at which the fixed part 71 of the fastening tab 70 is attached, and the site, i.e., a second inelastic area 38C (see Fig.4) may not be contracted. In this regard, the second inelastic area 38C may be disposed outside of the third elastic area 38 in the lateral direction X, while the fixed part 71 of the fastening tab 70 may be on the second inelastic area 38c. This ensures the fixing of the fixed part 71 and the ventral region 13.

Moreover, the pair of fastening tabs 70 may be disposed laterally outward of the third elastic areas 38 in which the middle elastic members 41C are arranged, and the fastening tab 70 is pulled laterally outward under a contractile force of the middle elastic members 41C. Thus, the engageable area 73 bites into a receiving area 78 so that a connecting means can be prevented from easily disconnecting.

The fastening elements of the engageable area 73 may be releasably engageable with the exterior sheet 27 formed of fibrous nonwoven fabrics defining the body non-facing surface of the dorsal region 14. In this regard, the diaper 10 may include a connecting means 79 whereby the lateral edges 16c, 16d of the ventral region 13 and the lateral edges 17c, 17d of the dorsal region 14 may respectively be releasably engageable with each other. To stably engage the fastening elements of the engageable area 73 with the dorsal region 14, a receiving sheet/landing sheet composed of loop elements of the mechanical fastener may be disposed on the associated part of the dorsal region 14. Moreover, when the exterior sheet 27 is constructed of multiple sheets of which an exteriormost sheet is formed of plastic films, a sensitive pressure adhesive layer is preferable to be used instead of the fastening elements on the hook sheet of the engageable area 73. The fixed part 71 of the fastening tab 70 may be fixed between the interior and exterior sheets 25, 27 without fixing on the body non-facing surface of the dorsal region 14, or may be fixed on the body facing surface of the dorsal region. A gripper part of the fastening tab 70 extending laterally outward from the engageable area 73 is preferable to have a size such as the gipping is easy and the fingers of a caretaker does not come in contact with the engageable area 73 when the caretaker grips the gripper part. The fastening tab 70 may be in more than one form at one side of the ventral region 13. Moreover, the fastening tab 70 may be elastically stretchable, attached in more than one folded and extendable state, or have the free part 73 dividable in more than one form, provided with the respective engageqable area 73.

The pair of fastening tabs 70 may each be disposed nearer to the upper edge 16b of the ventral region 13 than the lower edge 16a of the ventral region 13. This makes it possible to prevent the upper thighs of the hind legs 7 from being excessively tightened by the fastening tabs 70. In addition, as a tensile force of the fastening tabs 70 do not directly exert the lower edge area 33 of the ventral region 13 from the positional relationship of the fastening tabs 70, the upper thighs of the hind legs 7 should not be excessively tightened by the lower edge area 33 of the ventral region 13, and even if the lower edge area 33 has abutted against the hind legs 7 during the dog's walking, the lower edge area 33 is deformable in response to the movements of the hind leg 7 and should not create a discomfort feeling against the dog.

Referring to Fig. 2, the intermediate region 15 of the diaper 10 may be formed with a generally U-shaped cutting line 80 (having an open end and closed end) for a tail opening through which the tail 6 of the dog 5 is passed. The cutting line 80 may be positioned between the distal edges 56a of the containment flaps 52 and may penetrate the bodyside liner 61, the liquid barrier sheet 62 and the cover sheet 50 in planar view of the diaper 10. When putting the diaper 10 on the dog 5, the ventral region 13 and the dorsal region 14 can easily be connected to each other by engaging the engageable area 73 of the fastening tab 70 with the receiving area 78 of the dorsal region 14, with the tail 6 passed through the tail opening formed by the cutting line 80. The absorbent core 60 may spacedly be positioned from a vicinity of the open end of the cutting line 80 to the side of the ventral region 13. Thus, the absorbent core 60 is not present around the cutting line 80.

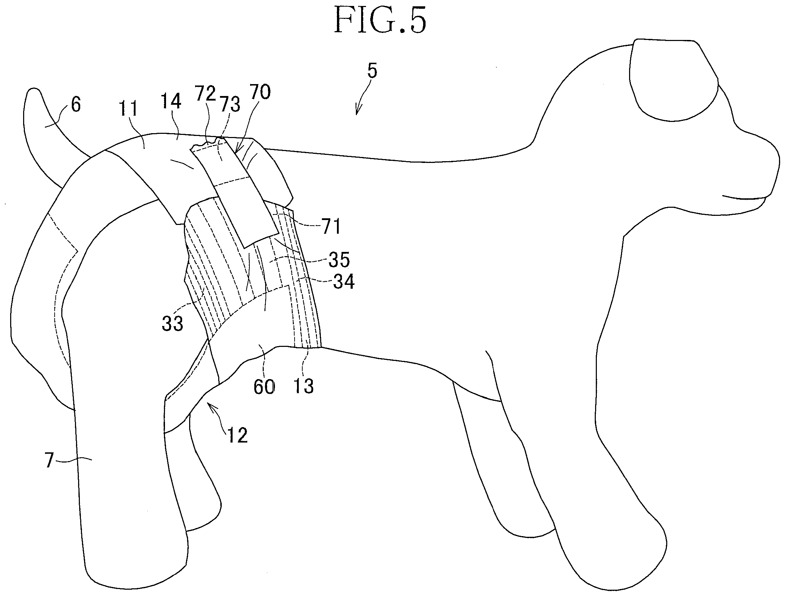

Referring to Figs. 3 and 5, when the diaper 10 is put on the hindquarter of the dog 5, the lateral edges 16c, 16d of the ventral region 13 and the lateral edges 17c, 17d of the dorsal region 14 may be connected to each other, with the engageable areas 73 of the fastening tabs 70 in the ventral region 13 engaged with the receiving areas 78 in the dorsal region 14, and with a loin opening 21 and a pair of leg opening 22 formed (see Fig. 1) . According to this embodiment, the diaper 10 being provided with the connecting means 70 for releasably engaging the lateral edges 16c, 16d of the ventral region 13 and the lateral edges 17c, 17d of the dorsal region 14, it is possible to put the diaper 10 on dogs having different loin sizes, for example, from a dog having a relatively slim body to a dong having a relatively chubby body. The lower edge area 33 of the ventral region 13 is defined by the first elastic area 36 contractible in the lateral direction X, and the lower edge area 33 can be abutted against the body of the dog, so that the diaper 10 put on the dog 5 can be prevented from displacing the position. In addition, the tensile stress of the first elastic area 36 being stronger than the tensile stress of the second elastic area 37, even when the dog 5 moves back and forth the hind legs 7 during walking, the lower edge area 33 can stably be abutted against the dog's body by the tensile stress of the first elastic area 36. Moreover, the lower edge area 33 being elastically stretchable and contractible, even if the lower edge area 33 is abutted against the hind legs 7 during the dog's walking, the lower edge area 33 is deformable in response to the movements of the hind legs 7, and should not create a discomfort feeling against the dog 5. Although the dog 5 does not have a large constricted part on its body, the diaper 10 put on the dog 5 can reliably be prevented from displacing the position by the tensile stress of the second elastic area 37. The tensile stress of the second elastic area 37 being stronger than the tensile stress of the third elastic area 38, body exudates such as urine can be prevented from leaking out of the loin opening 21.

In addition, the dorsal region 14 having the elastic areas 36, 37, 38 contractible in the lateral direction X, the diaper 10 also has the dorsal region 14 abutted against the dog's body by tensile stresses of the elastic areas. This makes it possible to enlarge areas to be abutted against the dog's body, and the diaper 10 put on the dog 5 can reliably be prevented from displacing the position.

Referring again to Fig. 2, the diaper10 may be configured by a rectangular ventral panel 16, a rectangular dorsal panel 17 and a rectangular absorbent panel 12. A lower edge 16a of the ventral region 13 may extend linearly in the lateral direction X, both lateral edges of the intermediate region 15(the absorbent panel 12) may extend linearly in the longitudinal direction, and they may cross each other. If the lower edge 16a and/or both lateral edges of the absorbent panel cross each other in a curved state, the curved parts may be abutted against the upper thighs of the hind legs 7 during the dog's walking, and the walking may be interfered. However, in the diaper 10 according to the present embodiment, the ventral and dorsal panels 16, 17 being orthogonal to the absorbent panel 12, the ventral panel 16 and the absorbent panel 12 are not positioned outside of the upper thighs of the hind legs 7. Thus, when the hind legs 7 move back and forth, the dog's walking is not interfered by abutting against part of the diaper 10. In addition, the diaper 10 may be configured by the three panels, and it may be easy to be manufactured.

### <Second Embodiment>

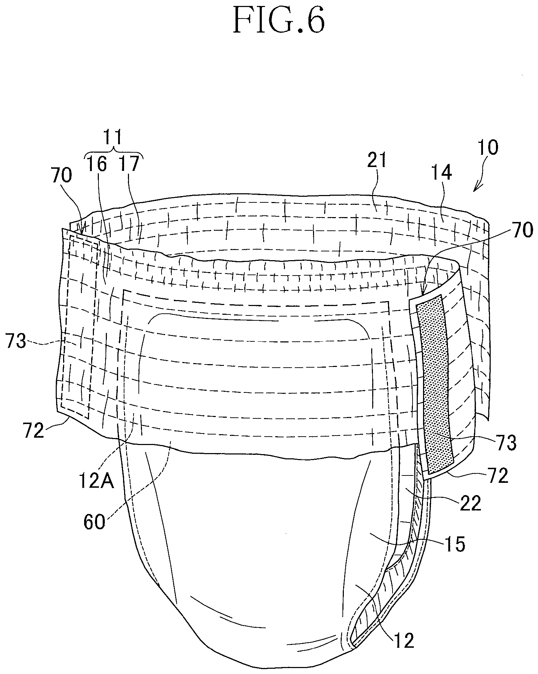

Referring to Fig. 6, a diaper 10 according to the present embodiment has a basic configuration similar to the diaper 10 according to the first embodiment unless otherwise noted, the structure of the latter is applied to that of the former, and the former will be described only for points different from the latter.

In the diaper 10 according to the present embodiment, a pair of engageable areas 73 of fastening tabs 70 may be disposed on the body facing surface at both lateral edges 16c, 16d of the ventral region 13, extending in the longitudinal direction Y from the lower edge 16a to the upper edge 16b. According to the diaper 10, if only at least part of each engageable area 73 extending in the longitudinal direction Y and at least part of the receiving area 78 are engaged with each other, both lateral edges 16c, 16c of the ventral region 13 and both lateral edges 17c, 17d of the dorsal region 14 can be engaged with each other. This makes it possible to easily put the diaper 10 on the dog 5.

In the first and second embodiments, although the diaper 10 is configured by three separate panels, i.e., the absorbent panel 12, the ventral panel 16 and the dorsal panel 17, the ventral and dorsal region 13, 14 may be formed by a single annular panel having no seams, and a base sheet of the ventral, dorsal and intermediate regions 13, 14, 15 may be formed by a continuous single chassis having no seams.

The components of the diaper 10 are not limited to those to the present specification but other various types of material widely used in the relevant to technical field may be used without limitation unless otherwise stated. Terms such as "first" and "second" used in the specification and claims are used merely to distinguish the similar elements, similar positions or similar means.

### [Description of Reference Signs]

5 pet (dog)
6 tail
10 absorbent article (diaper)
12 absorbent panel
13 ventral region
14 dorsal region
15 intermediate region
16 ventral panel
16a lower edge
16b upper edge
16c lateral edge
16d lateral edge
17 dorsal panel
17a lower edge
17b upper edge
17c lateral edge
17d lateral edge
25 interior sheet (interior surface sheet)
27 exterior sheet(exterior surface sheet)
33 lower edge area
34 upper edge area
35 middle area
36B inelastic area
36 first elastic area
37 second elastic area
38B second inelastic area
38 third elastic area
41 first elastic member
41A lower elastic member
41B upper elastic member
41C middle elastic member
60 absorbent core
70 fastening tab
71 proximal edge
72 distal edge
73 receiving area
79 connecting means
X lateral direction
Y longitudinal direction

## Claims

1. An absorbent article (10) for a pet animal (5) having a longitudinal direction (Y) and a lateral direction (X), and including a ventral region (13), a dorsal region (14) and an intermediate region (15) extending in the longitudinal direction between the ventral region and the dorsal region and including an absorbent core (60) extending from the intermediate region to the ventral region, wherein:
the ventral region (13) and the dorsal region (14) each have a lower edge (16a, 17a) and an upper edge (16b, 17b) both extending in the lateral direction (X) and opposed to each other in the longitudinal direction, and the ventral region (13) and the dorsal region (14) both have lateral edges (16c,16d,17c,17d) extending in the longitudinal direction between the lower edges and the upper edges, the ventral region (13) has a lower edge area (33) including the lower edge (16a,17a), an upper edge area (34) including the upper edge (16b,17b), a middle area (35) positioned between the lower edge area and the upper edge area, the absorbent article further having a connecting means (79) for detachably connect both lateral edges of the ventral region (13) and both lateral edges of the dorsal region (14) to each other;
the intermediate region (15) includes a tail opening passable through a tail (6) of the pet animal (5);
the absorbent core (60) is positioned between the tail opening and the ventral region (13);
the lower edge area (33) is defined by an elastic area (36) contractible in the lateral direction (X);
wherein the absorbent article (10) has a body facing surface to face a pet animal's body and a body non-facing surface opposite to the body facing surface;
the ventral region (13) includes an interior sheet (25) lying on the body facing surface, an exterior sheet (27) lying on the body non-facing surface, and thread-like or string-like elastic members (41) extending in the lateral direction between the interior sheet and the exterior sheet;
the elastic area (36) is a first elastic area defined by the elastic members (41A) located on the side of the lower edges (16a,17a), and the second elastic upper edge-area (37) is a second elastic area-defined by the elastic members (41B) located on the side of the upper edges (16b, 17b),
wherein a tensile stress of the first elastic area is stronger than a tensile stress of the second elastic area,
wherein a loin opening (21) and a pair of leg openings (22) are respectively defined when both lateral edges of the ventral region (13) and both the lateral edges of the dorsal region (14) has been connected to each other;
the first elastic area is defined by both elastic areas (36A) spaced apart from each other in the lateral direction (X), and an inelastic area (36B) is defined by an area overlapping with the absorbent core (60) in planar view between both elastic areas (36A); and
the middle area (35) is defined by a third elastic area (38) adjacent to the first and second elastic areas (36,37) and contractible in the lateral direction (X),
wherein the connecting means (79) includes a pair of fastening tabs (70) extending outward in the lateral direction from both lateral edges (16c, 16d) of the ventral region (13), and receiving areas (73) positioned on the body non-facing surface of the dorsal region (14) and releasably engageable with the respective fastening tabs (70); and
the pair of fastening tabs (70) are disposed outward in the lateral direction of the third elastic area (38).

2. The absorbent article (10) according to claim 1 further including a rectangular ventral panel (16) forming the ventral region, a rectangular dorsal panel (17) forming the dorsal region, and a rectangular absorbent panel (12) including an absorbent core (60) and extending in the longitudinal direction (X) while connecting to the ventral region and the dorsal region; and
the lower edge (16a) of the ventral region extend linearly in the lateral direction, both lateral edges of the intermediate region (15) extend in the longitudinal direction, and both the lateral edges of the intermediate region (15) and the lower edge (16a) of the ventral region orthogonally intersect with each other.

3. The absorbent article (10) according to claim 1, wherein the pair of fastening tabs (70) are disposed nearer to the upper edge (16b) than the lower edge (16a) of the ventral region (13).

4. The absorbent article according to any one of claims 1 through 3 wherein the dorsal region (14) has an elastic area contractible in the lateral direction.

## Patentansprüche

1. Saugfähiger Artikel (10) für ein Haustier (5), der eine Längsrichtung (Y) und eine Seitenrichtung (X) aufweist und eine ventrale Region (13), eine dorsale Region (14) und eine Zwischenregion (15), die sich in der Längsrichtung zwischen der ventralen Region und der dorsalen Region erstreckt und einen saugfähigen Kern (60) beinhaltet, der sich von der Zwischenregion zu der ventralen Region erstreckt, beinhaltet, wobei:
die ventrale Region (13) und die dorsale Region (14) jeweils eine Unterkante (16a, 17a) und eine Oberkante (16b, 17b) aufweisen, die sich beide in der Seitenrichtung (X) und einander gegenüberliegend in der Längsrichtung erstrecken, und die ventrale Region (13) und die dorsale Region (14) beide Seitenkanten (16c, 16d, 17c, 17d) aufweisen, die sich in der Längsrichtung zwischen den Unterkanten und den Oberkanten erstrecken, wobei die ventrale Region (13) einen Unterkantenbereich (33), der die Unterkante (16a, 17a) beinhaltet, einen Oberkantenbereich (34), der die Oberkante (16b, 17b) beinhaltet, und einen Mittelbereich (35) aufweist, der zwischen dem Unterkantenbereich und dem Oberkantenbereich positioniert ist, wobei der saugfähige Artikel ferner ein Verbindungsmittel (79) zum lösbaren Verbinden beider Seitenkanten der ventralen Region (13) und beider Seitenkanten der dorsalen Region (14) miteinander aufweist;
die Zwischenregion (15) eine Schwanzöffnung beinhaltet, durch die ein Schwanz (6) des Haustiers (5) durchgeführt werden kann;
der saugfähige Kern (60) zwischen der Schwanzöffnung und der ventralen Region (13) positioniert ist;
der Unterkantenbereich (33) durch einen elastischen Bereich (36) definiert ist, der in der Seitenrichtung (X) zusammenziehbar ist;
wobei der saugfähige Artikel (10) eine dem Körper zugewandte Oberfläche, um dem Körper des Haustiers zugewandt zu sein, und eine dem Körper abgewandte Oberfläche entgegengesetzt der dem Körper zugewandten Oberfläche aufweist;
die ventrale Region (13) ein Innenblatt (25), das auf der dem Körper zugewandten Oberfläche aufliegt, ein Außenblatt (27), das auf der dem Körper abgewandten Oberfläche aufliegt, und fadenförmige oder schnurartige elastische Elemente (41) beinhaltet, die sich in der Seitenrichtung zwischen dem Innenblatt und dem Außenblatt erstrecken;
der elastische Bereich (36) ein erster elastischer Bereich ist, der durch die elastischen Elemente (41A) definiert ist, die sich auf der Seite der Unterkanten (16a, 17a) befinden, und der zweite elastische Oberkantenbereich (37) ein zweiter elastischer Bereich ist, der durch die elastischen Elemente (41B) definiert ist, die sich auf der Seite der Oberkanten (16b, 17b) befinden,
wobei eine Zugspannung des ersten elastischen Bereichs stärker als die Zugspannung des zweiten elastischen Bereichs ist,
wobei eine Lendenöffnung (21) und ein Paar Beinöffnungen (22) jeweils definiert sind, wenn beide Seitenkanten der ventralen Region (13) und beide der Seitenkanten der dorsalen Region (14) miteinander verbunden wurden;
der erste elastische Bereich durch beide elastischen Bereiche (36A) definiert ist, die voneinander in der Seitenrichtung (X) beabstandet sind, und ein unelastischer Bereich (36B) durch einen Bereich definiert ist, der mit dem saugfähigen Kern (60) in planarer Ansicht zwischen beiden elastischen Bereichen (36A) überlappt; und
der Mittelbereich (35) durch einen dritten elastischen Bereich (38) angrenzend an den ersten und zweiten elastischen Bereich (36, 37) und zusammenziehbar in der Seitenrichtung (X) definiert ist,
wobei das Verbindungsmittel (79) ein Paar Befestigungslaschen (70), das sich nach außen in der Seitenrichtung von beiden Seitenkanten (16c, 16d) der ventralen Region (13) erstreckt, und Empfangsbereiche (73) beinhaltet, die auf der dem Körper abgewandten Oberfläche der dorsalen Region (14) positioniert sind und mit den jeweiligen Befestigungslaschen (70) lösbar eingreifbar sind; und
das Paar Befestigungslaschen (70) nach außen in der Seitenrichtung des dritten elastischen Bereichs (38) angeordnet ist.

2. Saugfähiger Artikel (10) nach Anspruch 1, ferner beinhaltend eine rechteckige ventrale Stoffbahn (16), die die ventrale Region ausbildet, eine rechteckige dorsale Stoffbahn (17), die die dorsale Region ausbildet, und eine rechteckige saugfähige Stoffbahn (12), die einen saugfähigen Kern (60) beinhaltet und sich in der Längsrichtung (X) beim Verbinden mit der ventralen Region und der dorsalen Region erstreckt; und
wobei sich die Unterkante (16a) der ventralen Region linear in der Seitenrichtung erstreckt, sich beide Seitenkanten der Zwischenregion (15) in der Längsrichtung erstrecken und sich sowohl die Seitenkanten der Zwischenregion (15) als auch die Unterkante (16A) der ventralen Region einander rechtwinklig überschneiden.

3. Saugfähiger Artikel (10) nach Anspruch 1, wobei das Paar Befestigungslaschen (70) näher an der Oberkante (16b) als der Unterkante (16a) der ventralen Region (13) angeordnet ist.

4. Saugfähiger Artikel nach einem der Ansprüche 1 bis 3, wobei die dorsale Region (14) einen elastischen Bereich aufweist, der in der Seitenrichtung zusammenziehbar ist.

## Revendications

1. Article absorbant (10) pour un animal de compagnie (5) ayant une direction longitudinale (Y) et une direction latérale (X), et comportant une région ventrale (13), une région dorsale (14) et une région intermédiaire (15) s'étendant dans la direction longitudinale entre la région ventrale et la région dorsale et comportant un noyau absorbant (60) s'étendant à partir de la région intermédiaire vers la région ventrale, dans lequel :
la région ventrale (13) et la région dorsale (14) ont chacune un bord inférieur (16a, 17a) et un bord supérieur (16b, 17b) s'étendant tous les deux dans la direction latérale (X) et opposés l'un à l'autre dans la direction longitudinale, et la région ventrale (13) et la région dorsale (14) ont toutes les deux des bords latéraux (16c, 16d, 17c, 17d) s'étendant dans la direction longitudinale entre les bords inférieurs et les bords supérieurs, la région ventrale (13) a une zone de bord inférieur (33) comportant le bord inférieur (16a, 17a), une zone de bord supérieur (34) comportant le bord supérieur (16b, 17b), une zone médiane (35) positionnée entre la zone de bord inférieur et la zone de bord supérieur, l'article absorbant ayant en outre un moyen de liaison (79) pour relier de manière détachable les deux bords latéraux de la région ventrale (13) et les deux bords latéraux de la région dorsale (14) les uns aux autres ;
la région intermédiaire (15) comporte une ouverture de queue pouvant être traversée par la queue (6) de l'animal de compagnie (5) ;
le noyau absorbant (60) est positionné entre l'ouverture de queue et la région ventrale (13);
la zone de bord inférieur (33) est définie par une zone élastique (36) pouvant se contracter dans la direction latérale (X) ;
dans lequel l'article absorbant (10) a une surface tournée vers le corps pour faire face au corps d'un animal de compagnie et une surface non tournée vers le corps opposée à la surface tournée vers le corps ;
la région ventrale (13) comporte une feuille intérieure (25) reposant sur la surface tournée vers le corps, une feuille extérieure (27) reposant sur la surface non tournée vers le corps, et des éléments élastiques de type fil ou de type corde (41) s'étendant dans la direction latérale entre la feuille intérieure et la feuille extérieure ;
la zone élastique (36) est une première zone élastique définie par les éléments élastiques (41A) situés sur le côté des bords inférieurs (16a, 17a), et la deuxième zone de bord supérieur élastique (37) est une deuxième zone élastique définie par les éléments élastiques (41B) situés sur le côté des bords supérieurs (16b, 17b),
dans lequel une contrainte de traction de la première zone élastique est plus forte qu'une contrainte de traction de la deuxième zone élastique,
dans lequel une ouverture de rein (21) et une paire d'ouvertures de patte (22) sont respectivement définies lorsque les deux bords latéraux de la région ventrale (13) et les deux bords latéraux de la région dorsale (14) ont été reliés les uns aux autres ;
la première zone élastique est définie par les deux zones élastiques (36A) espacées l'une de l'autre dans la direction latérale (X), et une zone inélastique (36B) est définie par une zone chevauchant le noyau absorbant (60) en vue plane entre les deux zones élastiques (36A) ; et
la zone médiane (35) est définie par une troisième zone élastique (38) adjacente aux première et deuxième zones élastiques (36, 37) et pouvant se contracter dans la direction latérale (X),
dans lequel le moyen de liaison (79) comporte une paire de languettes de fixation (70) s'étendant vers l'extérieur dans la direction latérale à partir des deux bords latéraux (16c, 16d) de la région ventrale (13), et des zones de réception (73) positionnées sur la surface non tournée vers le corps de la région dorsale (14) et pouvant venir en prise de manière amovible avec les languettes de fixation (70) respectives ; et
la paire de languettes de fixation (70) sont disposées vers l'extérieur dans la direction latérale de la troisième zone élastique (38).

2. Article absorbant (10) selon la revendication 1, comportant en outre un panneau ventral rectangulaire (16) formant la région ventrale, un panneau dorsal rectangulaire (17) formant la région dorsale, et un panneau absorbant rectangulaire (12) comportant un noyau absorbant (60) et s'étendant dans la direction longitudinale (X) tout en étant relié à la région ventrale et à la région dorsale ; et
le bord inférieur (16a) de la région ventrale s'étend linéairement dans la direction latérale, les deux bords latéraux de la région intermédiaire (15) s'étendent dans la direction longitudinale, et les deux bords latéraux de la région intermédiaire (15) et le bord inférieur (16a) de la région ventrale se croisent perpendiculairement.

3. Article absorbant (10) selon la revendication 1, dans lequel la paire de languettes de fixation (70) sont disposées plus près du bord supérieur (16b) que du bord inférieur (16a) de la région ventrale (13).

4. Article absorbant selon l'une quelconque des revendications 1 à 3, dans lequel la région dorsale (14) a une zone élastique pouvant se contracter dans la direction latérale.
